(19) Europäisches Patentamt
European Patent Office
Office européen des brevets

(11) Veröffentlichungsnummer: **0 136 730**
**B1**

(12) **EUROPÄISCHE PATENTSCHRIFT**

(45) Veröffentlichungstag der Patentschrift: **27.04.88**

(51) Int. Cl.⁴: **C 07 D 213/74**, C 07 D 401/12 // A61K7/13

(21) Anmeldenummer: **84200969.8**

(22) Anmeldetag: **04.07.84**

(54) **2-Aminonitropyridinderivate und Verfahren zu ihrer Herstellung.**

(30) Priorität: **21.09.83 DE 3334029**

(43) Veröffentlichungstag der Anmeldung: **10.04.85 Patentblatt 85/15**

(45) Bekanntmachung des Hinweises auf die Patenterteilung: **27.04.88 Patentblatt 88/17**

(84) Benannte Vertragsstaaten: **CH DE FR GB IT LI**

(56) Entgegenhaltungen:
**EP-A-0 137 524**
**DE-A-1 949 750**
**DE-A-2 334 401**
**DE-B-1 695 637**

**CHEMICAL ABSTRACTS, Band 41, Nr. 5, 10. März 1947, Columbus, Ohio,USA, A. MANGINI et al. "Aromatic nitro derivatives",Spalten 1224f-1225b**
**CHEMICAL ABSTRACTS, Band 98, Nr. 13, 28. März 1983, Columbus, Ohio,USA, P. AHUJA et al. "Lactam acetals",Seite 584, Spalte 1, Zusammenfassung Nr. 107 106q**

(73) Patentinhaber: **RÜTGERSWERKE AKTIENGESELLSCHAFT, Mainzer Landstrasse 217, D-6000 Frankfurt am Main 1 (DE)**

(72) Erfinder: **Orth, Winfried, Dr., Am Schachtelgraben 28, D-6733 Hassloch/Pfalz (DE)**
Erfinder: **Fickert, Werner, Dr., Stockacher Strasse 14, D-6800 Mannheim 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 136 730 B1

LIBER, STOCKHOLM 1988

**Beschreibung**

Aus DE-A-1 949 750 ist die Verwendung bestimmter Aminonitropyridinderivate als direktziehende Haarfärbemittel bekannt. Aus DE-B-1 695 637 sind ferner 2-Amino-5-nitropyridine bekannt, die sich durch Reduktion der 5-Nitrogruppe in Haarfärbemittel überführen lassen.

Die Erfindung betrifft neue Nitropyridinderivate, die in 2-Stellung eine primäre, sekundäre oder tertiäre Aminogruppe haben. Diese Verbindungen stellen einerseits wichtige pharmazeutische Zwischenprodukte dar, andererseits können sie überraschenderweise als nichttoxische Kupplerkomponenten in Oxidationshaarfarben verwendet werden und ergeben brillante Farbtönungen.

Die erfindungsgemäßen 2-Aminonitropyridinderivate entsprechen der allgemeinen Formel I

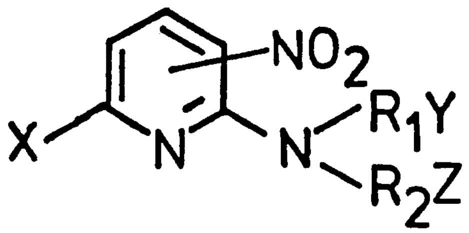

(I)

wobei die Nitrogruppe in 3- oder 5-Stellung steht, X eine Alkoxygruppe mit 1 - 3 C-Atomen bedeutet und $R_1$ und $R_2$ gleich oder verschieden sind und Wasserstoff, einen Cyclopropyl- oder einen Alkyl- oder Alkenylrest mit 1 - 3 C-Atomen, einen Phenyl- oder einen Pyrrolylrest darstellen, wobei diese Reste als Substituenten eine Hydroxyl- oder eine Aminogruppe der allgemeinen Formel II

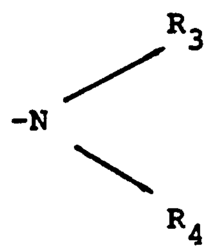

(II)

tragen können, worin $R_3$ und $R_4$ gleich oder verschieden sind und Wasserstoff oder einen unsubstituierten oder an beliebiger Stelle hydroxyl- oder aminosubstituierten Alkylrest mit höchstens 3 C-Atomen bedeuten.

Diese Derivate sind bei Raumtemperatur gegen Oxidation durch Luftsauerstoff weitgehend stabil. Sie sind in Wasser bzw. in einem Gemisch aus Wasser und einem Lösungsvermittler wie z. B. Alkohol löslich.

X kann eine Methoxy-, Äthoxy-, Propoxy- oder i-Propoxygruppe darstellen.

Die Aminogruppe in 2-Stellung kann eine primäre, sekundäre oder tertiäre Aminogruppe sein. Ist diese Aminogruppe sekundär oder tertiär, können die Reste $R_1$ und $R_2$ gleich oder verschieden sein und ausser Wasserstoff z. B. einen Methyl-, Äthyl-, Propyl-oder i-Propylrest, einen Phenylrest oder einen an einem C-Atom verknüpften Pyrrolylrest bedeuten. Diese Reste können ihrerseits als Substituenten eine Hydroxyl- oder eine Aminogruppe der Formel II tragen. Beispiele für derart substituierte Reste $R_1$ oder $R_2$ sind Hydroxymethyl-, Hydroxyäthyl- oder Hydroxypropyl.

Stelle hydroxyl- oder aminsubstituierten Alkylrest mit bis zu 3 C-Atomen bedeuten.

Beispiele für die Aminogruppe der Formel II sind die unsubstituierte Aminogruppe selbst sowie die Methyl-, Dimethyl-, Äthyl-, Diäthyl-, Methyläthyl-, Propyl-, i-Propyl-, Dipropyl-, Diisopropyl-, Aminomethyl-, Aminoäthyl-, Aminopropyl-, Bis-aminomethyl-, Bisaminoäthyl-, Bis-aminopropyl, Hydroxymethyl-, Dihydroxymethyl-, Hydroxyäthyl-, Dihydroxyäthyl-, Hydroxipropyl- oder Dihydroxypropylaminogruppe oder ein Pyrrolyl- oder Methylpyrrolylrest.

Die erfindungsgemäßen 2-Aminonitropyridinderivate werden dargestellt durch Umsetzung der entsprechenden Nitropyridine, die in 2-Stellung einen durch Amine substituierbaren Rest besitzen, mit den jeweiligen Aminen. Derartige substituierbare Reste sind Alkoxy- oder Sulfonsäuregruppen oder Halogene.

Diese Ausgangsnitropyridine werden bevorzugt durch Nitrieren der entsprechenden Alkoxi- bzw. Halogenalkoxipyridine erhalten, wie dies z. B. in der DE-A-3 308 449 beschrieben ist. Die Umsetzung dieser Nitropyridine mit den Aminen erfolgt bei mehrstündigem Behandeln der Reaktanden miteinander bei Normaldruck und Temperaturen im Bereich von 20 bis 100°C. Die Reaktanden werden dabei in einem polaren Lösungsmittel wie z. B. Wasser oder einem Alkohol intensiv miteinander vermischt. Sie sind in diesem Reaktionsmedium entweder gelöst oder ganz oder teilweise suspendiert oder emulgiert.

Die Reaktanden können in äquimolaren Mengen eingesetzt werden. Zum Erzielen einer besseren Ausbeute ist es jedoch ratsam, die billigere Aminkomponente im Oberschuß einzusetzen.

Die erhaltenen Endprodukte sind in kaltem Wasser unlöslich und können somit aus einem gekühlten wäßrigen oder mit Wasser versetzten Reaktionsmedium abgetrennt werden.

**Beispiel 1**

2-(2-Hydroxyphenylamino)-6-methoxy-3-nitropyridin

Eine Mischung aus 188,6 g (1 Mol) 2-Chlor-6-methoxy-3-nitro-pyridin und 262 g (2,4 Mol) o-Aminophenol in 500 ml Methanol wird allmählich bis zum Rückfluß erhitzt. Nach zweistündiger Erhitzungsdauer wird heiß abgesaugt, der Nutschenkuchen mit so viel Methanol gewaschen, bis das Filtrat praktisch farblos abläuft, und getrocknet. Es werden hellrote Kristalle vom Schmelzpunkt 206 - 7°C in einer Ausbeute von 94,6 % der Theorie erhalten.

**Beispiel 2**

2-[-3-(Dimethylaminopropyl)amino]-6-methoxy-3-nitropyridinhydrochlorid

Zu einer Suspension von 188,6 g (1 Mol) 2-Chlor-6-methoxy-3-nitropyridin in 300 ml Methanol werden unter Rühren und Kühlen 265,2 g (2,6 Mol) 3-(N,N-Dimethylamino)propylamin getropft. Die Mischung wird nach der Amin-Zugabe noch 2 Stunden nachgerührt und dann in 300 ml Wasser eingegossen, wobei die Substanz als gelbes Öl ausfällt. Das Öl wird abgetrennt und noch zweimal mit Wasser angerührt, bis die Wasserschicht nur noch schwach gelb gefärbt erscheint. Es wird in Isopropanol aufgenommen und durch Einleiten von HCl-Gas als HCl-Salz gefällt. Letzteres wird abgesaugt und mit Isopropanol gewaschen und getrocknet. Die Substanz wird in Form eines leuchtend gelb gefärbten Kristallisates mit dem Schmelzpunkt 186°C in einer Ausbeute von 76 % der Theorie erhalten.

**Beispiel 3**

6-Methoxy-3-nitro-2-n-propylaminopyridin

Zu einer gerührten und gekühlten (Wasserbad) Mischung aus 188,6 g (1 Mol) 2-Chlor-6-methoxy-3-nitropyridin und 200 ml Isopropanol werden in ca. 3 Stunden bei max. 25°C 141,9 g (2,4 Mol) n-Propylamin getropft. Es wird über Nacht weitergerührt, dann werden 1000 ml Wasser zugegeben, das ausgefallene Produkt wird abgesaugt, mit Wasser gewaschen und getrocknet. Die intensiv gelb gefärbte Titelverbindung vom Schmelzpunkt 69 - 70°C wird in einer Ausbeute von 98 % der Theorie erhalten.

**Beispiel 4**

2-(2-Hydroxyethylamino)-6-methoxy-3-nitropyridin

Zu einer Mischung aus 188,6 g (1 Mol) 2-Chlor-6-methoxy-3-nitropyridin mit 350 ml Isopropanol werden unter Rühren und Beibehaltung einer Temperatur von 25-30°C durch Kühlung 83,5 g (1,4 Mol) Aminoethanol eingetropft. Nach der Zugabe wird das Reaktionsgemisch noch 1 Stunde bei max. 30°C nachgerührt und dann in ca. 2 Stunden 47,5 ml 50-proz. Natronlauge bei max. 30°C zugetropft. Nach 6-stündigem Rühren werden 500 ml Wasser eingerührt und das Produkt wird abgesaugt, reichlich mit Wasser gewaschen und getrocknet. Die Verbindung wird in Form eines gelben Pulvers vom Schmelzpunkt 121 -2°C in einer Ausbeute von 95,5 % der Theorie erhalten.

**Beispiel 5**

2-(3-Hydroxyphenylamino)-6-methoxy-3-nitropyridin

Eine Mischung aus 188,6 g (1 Mol) 2-Chlor-6-methoxy-3-nitro-pyridin und 262 g (2,4 Mol) m-Aminophenol in 500 ml Methanol wird allmählich bis zum Rückfluß erhitzt. Nach zweistündiger Erhitzungsdauer wird die Mischung abgekühlt, 1000 ml Wasser eingerührt, die gebildeten Kristalle abgesaugt und gut mit Wasser gewaschen. Die Verbindung wird in Form organgefarbener Kristalle vom Schmelzpunkt 156-8°C in einer Ausbeute von 97,7 % der Theorie erhalten.

**Beispiel 6**

6-Ethoxy-2-methylamino-3-nitropyridin

In einer gerührten Mischung aus 202,6 g (1 Mol) 2-Chlor-6-ethoxy-3-nitropyridin und 500 ml Methanol werden unter Kühlung im Verlauf von 90 Min und bei 25 - 30°C Sumpftemperatur 202 g (2,6 Mol) 40-proz.

Monomethylamin-Lösung getropft. Anschließend wird die Mischung noch 5 Stunden nachgerührt, mit 650 ml Wasser versetzt, das Produkt abgesaugt, mit reichlich Wasser nachgewaschen und getrocknet. Fp. 106-7°C. Ausbeute: 183,2 g (92.9 % der Theorie).

**Patentansprüche**

1. 2-Aminonitropyridinderivate der allgemeinen Formel I

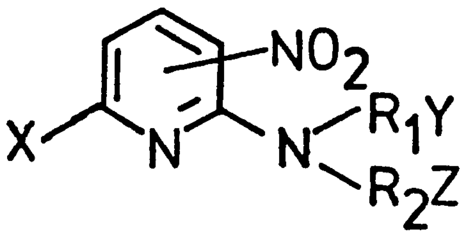

(I),

wobei die Nitrogruppe in 3- oder 5-Stellung steht, X eine Alkoxygruppe mit 1 - 3 C-Atomen bedeutet und $R_1$ und $R_2$ gleich oder verschieden sind und Wasserstoff, einen Cyclopropyl- oder einen Alkyl- oder Alkenylrest mit 1 - 3 C-Atomen, einen Phenyl- oder einen Pyrrolylrest darstellen, wobei diese Reste als Substienten eine Hydroxylgruppe oder eine Aminogruppe der allgemeinen Formel II

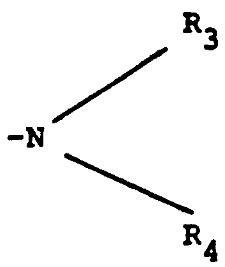

(II)

tragen können, worin $R_3$ und $R_4$ gleich oder verschieden sind und Wasserstoff, einen unsubstituierten oder an beliebiger Stelle hydroxyl- oder aminosubstituierten Alkylrest mit höchstens 3 C-Atomen bedeuten.

2. Verfahren zur Herstellung der 2-Aminonitropyridinderivate nach Anspruch 1, dadurch gekennzeichnet, daß in 2-Stellung halogen-, alkoxy- oder sulfonsäuresubstituierte Nitropyridine bei einer Temperatur im Bereich von 20 - 100°C mit den entsprechenden Aminen umgesetzt werden.

**Claims**

1. 2-aminonitropyridine derivatives of the general formula I

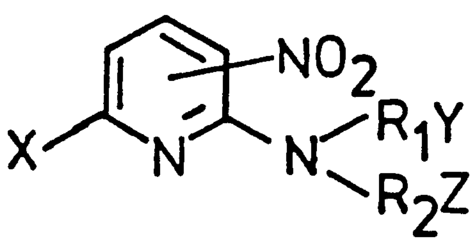

in which the nitro group is in the 3 or 5 position, X represents an alkoxy group with from 1 to 3 carbon atoms and $R_1$ and $R_2$ are the same or different and represent hydrogen, a cyclopropyl or an alkyl or alkenyl radical with from 1 to 3 carbon atoms, a phenyl or a pyrrolyl radical, it being possible for these radicals to carry as a substituent an hydroxyl group or an amino group of the general formula II

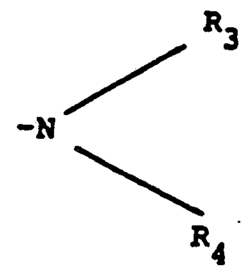

in which $R_3$ and $R_4$ are the same or different and represent hydrogen, an unsubstituted alkyl radical with at most 3 carbon atoms or an alkyl radical with at most 3 carbon atoms which is hydroxyl- or aminosubstituted at any position.

2. A process for the preparation of 2-aminonitropyridine derivatives according to Claim 1, characterized in that nitropyridines halogen-, alkoxy- or sulphonic acid-substituted in the 2-position are reacted at a temperature in the range of from 20 to 100° C with the corresponding amines.

**Revendications**

1. Dérivés de 2-aminonitropyridine de formule générale I

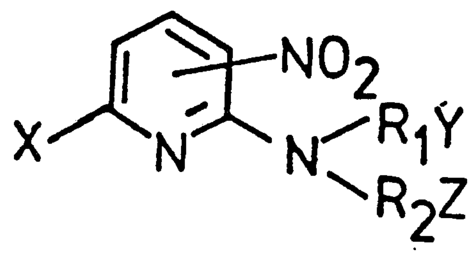

(I)

dans laquelle le groupe nitro est situe en position 3 ou 5, dans laquelle X représente un groupe alcoxy ayant 1-3 atomes de carbone et dans laquelle $R_1$ et $R_2$ qui sont identiques ou différents représentent l'hydrogène, un radical cyclopropyle ou alcoyle ou alcényle ayant 1-3 atomes de carbone, un radical phényle ou un radical pyrrolyle, ces radicaux pouvant porter en tant que substituants un groupe hydroxyle ou un groupe amino de formule générale II

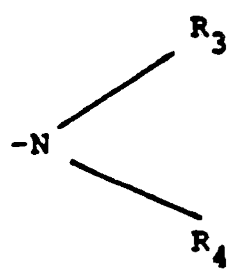

(II)

dans laquelle $R_3$ et $R_4$ qui sont identiques ou différents, représentent l'hydrogène, un radical alcoyle non substitué ou hydroxyl- ou amino-substitué en n'importe quel endroit avec au maximum 3 atomes de carbone.

2. Procédé pour la préparation de dérivés de 2-aminonitropyridine selon la revendication 1, caractérisé en ce que l'on fait réagir des nitropyridines substituées en position 2 par des radicaux halogène ou alcoxy ou par l'acide sulfonique à une température située dans le domaine de 20-100° C à l'aide des amines correspondantes.